# EUROPÄISCHE PATENTANMELDUNG

(11) **EP 0 729 987 A2**
(43) Veröffentlichungstag der Anmeldung: **04.09.1996**
(21) Anmeldenummer: 96102837.0
(22) Anmeldetag: 26.02.1996
(51) Int. Cl.: C08F 220/12, A61K 7/06, C08F 218/04

(54) **Für den Einsatz in Haarfestigungsmitteln geeignete sulfogruppenhaltige Polymere**

(30) Priorität: 02.03.1995 DE 19507249
(71) Anmelder: BASF AKTIENGESELLSCHAFT, 67056 Ludwigshafen (DE)
(72) Erfinder: Blankenburg, Rainer, Dr., 67067 Ludwigshafen (DE); Sperling, Karin, Dr., 67433 Neustadt (DE); Sanner, Axel, Dr., 67227 Frankenthal (DE)

(57) **Zusammenfassung**

Für wäßrige Haarfestigungsmittel geeignete Polymere, erhältlich durch radikalisch initiierte Lösungspolymerisation eines Monomerengemischs aus
A) 50 - 90 Gew.-% eines oder mehrerer Monomere aus der Gruppe der C₁-C₁₈-Alkylester der Acrylsäure oder der Methacrylsäure, der Vinylester von C₂-C₁₀-Monocarbonsäuren,
B) 10 - 25 Gew.-% eines sulfonsäuregruppenhaltigen Vinylmonomeren, und
C) 0 - 40 Gew.-% eines weiteren Monomeren.

## Beschreibung

Die vorliegende Erfindung betrifft für den Einsatz in wäßrigem Haarfestigungsmitteln geeignete Polymere, welche erhältlich sind durch radikalisch initiierte Lösungspolymerisation einer Monomerenmischung aus
A) 50 - 90 Gew.-% eines oder mehrerer Monomeren aus der Gruppe der C₁-C₁₈-Alkylester der Acrylsäure oder der Methacrylsäure, der Vinylester von C₂-C₁₀-Monocarbonsäuren,
B) 10 - 25 Gew.-% eines sulfonsäuregruppenhaltigen Vinylmonomeren, und
C) 0 - 40 Gew.-% eines weiteren Monomeren aus der Gruppe, bestehend aus Acrylsäure, Methacrylsäure, Acrylamid, Methacrylamid, C₃-C₈-N-Alkylacrylamide, N,N-Dimethylacrylamid, N-Vinylpyrrolidon und N-Vinylcaprolactam,

Weiterhin betrifft die Erfindung Haarfestigungsmittel enthaltend als Filmbildner solche Polymere.

Aufgrund der steigenden Anforderungen an die Umweltverträglichkeit auch von kosmetischen Produkten, besteht ein wachsender Bedarf an Haarkosmetikpolymeren, die sich für solche Zubereitungen eignen.

Aus der US-A 4 859 458 sind haarkosmetische Zubereitungen auf Basis von Copolymeren, enthaltend sulfonsäuregruppenhaltige Vinylmonomere, bekannt, wobei die Sulfonsäuregruppen als Salze von polyethoxilierten Aminen vorliegen. Diese Zubereitungen weisen vor allem konditionierende Eigenschaften auf.

In der EP-A 521 666, der EP-A 522 756 und der US-A 5 275 809 werden ampholytische Polymere aus Acrylamido-methylpropansulfonsäure und Diallyldimethylammoniumchlorid beschrieben, die in Conditionerformulierungen eingesetzt werden.

Aus der japanischen Offenlegungsschrift J 5 616 109 sind Tetrapolymere auf Acrylatbasis bekannt, welche neben sulfonsäuregruppenhaltigen Monomeren auch hydroxylgruppenhaltige Monomere enthalten. Zubereitungen dieser Polymere verleihen dem Haar einen eher weichen Griff bei weniger gut ausgeprägten festigenden Eigenschaften.

Aus der EP-A 611 566 sind Haarfestigeraerosole bekannt, welche Copolymere aus sulfonsäuregruppenhaltigen Vinylmonomeren und N-Monoalkyl-(meth)acrylamiden sowie gegebenenfalls weitere Acrylatmonomeren enthalten. Solche Copolymere sind wegen des relativ hohen Anteils an den teuren Sulfonat-Monomeren nicht nur aus wirtschaftlichen Gesichtspunkten wenig vorteilhaft, sie weisen aufgrund des starken Polyelektrolytcharakters auch relativ hohe Viskositäten in wäßriger Lösung bei eingeschränkter Verträglichkeit mit organischen Lösungsmitteln und einer gewissen Neigung zur Klebrigkeit auf.

Aufgabe der vorliegenden Erfindung war es, Polymer für den Einsatz in Haarfestigungsmitteln zu finden, welche neben guten festigenden Eigenschaften auch eine gute Wasserlöslichkeit bei gleichzeitig gutem Trocknungsverhalten aufweisen.

Demgemäß wurden die eingangs definierten Polymere sowie Haarfestigungsmittel, enthaltend solche Polymere, gefunden.

Geeignete Monomere A) sind erfindungsgemäß beispielsweise die C₁-C₁₈-Alkylester der Acrylsäure oder der Methacrylsäure, bevorzugt Ethylacrylat, tert.-Butylacrylat, Methylmethacrylat, Ethylmethacrylat, n-Butylmethacrylat, iso-Butylmethacrylat oder tert-Butylmethacrylat oder auch Dodecyl- oder Stearyl-acrylate und -methacrylate. Weiterhin kommen als Monomere A) auch die Vinylester von gesättigten C₂-C₁₀-Monocarbonsäuren in Betracht, insbesondere Vinylacetat und Vinylpropionat oder Vinylester langkettiger linearer oder verzweigter Fettsäuren wie beispielsweise der Versatic®-Säuren (Fa. Shell).

Es können auch Mischungen der Monomere A) eingesetzt werden. Vorzugsweise wird das oder die Monomere A) so gewählt, daß ein aus diesen bestehendes Homopolymer oder Copolymer eine Glasübergangstemperatur Tg von >10°C aufweisen würde. Die Monomere A werden in Mengen von 50 bis 90, vorzugsweise 70 bis 85 Gew.-% eingesetzt.

Als Monomere B) kommen erfindungsgemäß sulfonsäuregruppentragende Vinylmonomere in Betracht, beispielsweise 2-Acrylamid-2-methylpropansulfonsäure, 2-Methacrylamid-2-methyl-propansulfonsäure, 2-Acryloyl-ethansulfonsäure, 2-Methacryloylethansulfonsäure, 2-Acryloyl-propan-sulfonsäure, 2-Methacryloylpropansulfonsäure, Styrolsulfonsäure oder Vinylsulfonsäure. Bevorzugte Monomere B) sind 2-Acrylamid-2-methyl-propansulfonsäure, 2-Acrylsäure-2-methyl-propansulfonsäure oder 2-Methacrylsäure-2-methyl-propansulfonsäure.

Die Sulfonsäuregruppen der Monomeren B) werden vorzugsweise vor oder während der Polymerisation mit einer anorganischen Base oder einer organischen Stickstoff-Base mit 1 - 8 C-Atomen ganz oder teilweise in Salzform überführt. Als anorganische Basen eignen sich vor allem Alkalihydroxide wie Natrium- oder Kaliumhydroxid oder Erdalkalihydroxide wie Magnesiumhydroxid, Calciumhydroxid oder Bariumhydroxid oder auch Ammoniak. Als stickstoffbasische Verbindungen kommen Amine wie Methylamin, Dimethylamin, Ethylamin, Diethylamin und die entsprechenden höheren Homologen in Betracht oder, vorzugsweise Alkanolamine wie sie für die Kosmetik zugelassen sind, beispielsweise 2-Amino-2-methylpropanol oder Triethanolamin.

Die Monomere B) werden in Mengen von 10 bis 25, vorzugsweise 10 bis 20 Gew.-% eingesetzt.

Die erfindungsgemäßen Polymeren können weiterhin bis zu 40 Gew.-%, bevorzugt 0 bis 25 Gew.-%, an einem Monomeren ausgewählt aus der Gruppe bestehend aus Acrylsäure, Methacrylsäure, Acrylamid, Methacrylamid, C₃-C₈-N-Alkylacrylamiden, N,N-Dimethylacrylamid, N-Vinylpyrrolidon und N-Vinylcaprolactam, wobei die beiden letztgenannten Monomere bevorzugt sind, enthalten.

Die K-Werte betragen 15 bis 40, bevorzugt 20 bis 35. Die Bestimmung der K-Werte erfolgt nach H. Fikentscher, Cellulose-Chemie, 13 (1932), S. 58-64 und S. 71-74 gemessen in 1 gew.-%iger N-Methylpyrrolidon-Lösung.

Die Herstellung der erfindungsgemäßen Copolymerisate erfolgt nach der Methode der radikalischen Lösungspolymerisation in Alkoholen, gegebenenfalls in einem Gemisch aus Alkoholen und Wasser. Als Alkohole eignen sich C₁-C₄-Alkanole, wobei die in der Kosmetik üblicherweise verwendeten Alkohole Ethanol und Isopropanol bevorzugt sind.

Die Polymerisation erfolgt in Gegenwart geeigneter Mengen einer radikalbildenden Verbindung wie organischen Azo- oder Peroxo-Verbindungen als Polymerisationsinitiatoren.

Geeignete Initiatoren sind z.B. Diacylperoxide wie Dilauroyl-, Didecanoyl- und Dioctanoylperoxid, sowie Perester wie tert.-Butylperoxipivalat, tert.-Amylperoxipivalat oder tert.-Butylperoxineodecanoat, sowie Azoverbindungen wie Dimethyl-2,2-azobis(isobutyrat), 2,2-Azobis(isobutyronitril), 2,2-Azobis-(2-methyl-butyronitril) oder 2,2-Azobis-(2,2-dimethylvaleronitril).

Die Herstellung der Copolymerisate kann im Batch-Verfahren erfolgen, bevorzugt jedoch nach dem Zulaufverfahren, wobei sich ein Teil der Lösung des Monomerengemischs, in der Vorlage befindet und der restliche Teil über einen Zeitraum von mehreren Stunden zugefahren wird.

Es kann auch für den Reaktionsablauf vorteilhaft sein, das Lösungsmittel im Reaktor vorzulegen und die Monomeren, den Initiator und das Neutralisationsmittel zur Aufrechterhalten des erforderlichen pH-Wertes unter den Polymerisationsbedingungen zuzudosieren.

Die Dauer des Monomerenzulaufs wird üblicherweise so bemessen, daß die freigesetzte Polymerisationswärme unter den üblichen technischen Bedingungen gut abgeführt werden kann.

Die Polymerisation wird vorteilhafterweise bei Temperaturen von 60 bis 100°C, vorzugsweise 70 bis 85°C durchgeführt, wobei die Reaktion unter Eigendruck, Normaldruck oder unter Schutzgasüberdruck durchgeführt werden kann. Als Schutzgas eignet sich beispielsweise Stickstoff.

Falls die Polymere in rein wäßrigen Formulierungen eingesetzt werden sollen oder Lösungsmittel wie beispielsweise Methanol verwendet werden, empfiehlt sich ein Austausch des organischen Lösungsmittels gegen Wasser. Der Austausch von organischen Lösungsmitteln gegen Wasser erfolgt üblicherweise durch Zugabe der entsprechenden Wassermenge zum Reaktionsansatz nach erfolgter Polymerisation und gleichzeitigem oder nachfolgendem Abdestillieren des Lösungsmittels bei Normaldruck oder bei vermindertem Druck. Besonders günstig ist die Abtrennung des organischen Lösungsmittels durch Wasserdampfdestillation.

Der pH-Wert des Polymerisationsgemischs wird vorzugsweise so eingestellt, daß die Estergruppen der Monomere gegen saure Hydrolyse oder Umesterung stabil sind, wobei die Einstellung mit den bereits erwähnten anorganischen oder organischen Basen erfolgen kann. Eine besonders einfache Vorgehensweise besteht darin, die Monomere B) als wäßrige Lösung mit einem pH-Wert von mindestens 4 der Polymerisationszone zuzuführen. Man kann jedoch die Monomere B) in nicht neutralisierter Form dem Reaktionsgemisch zufügen und gleichzeitig entsprechende Mengen einer Base zufügen, um den pH-Wert in den gewünschten Bereich zu bringen.

Die wäßrigen oder wäßrig-alkoholischen Polymerlösungen enthalten die Polymere üblicherweise in Mengen von 10 bis 70, vorzugsweise 15 bis 50 Gew.-%.

Als Trocknungsverfahren zur Erzeugung pulverförmiger Polymerisate kommen alle solche in Frage, die zur Trocknung aus wäßriger Lösung geeignet sind. Bevorzugte Verfahren sind die Sprühtrocknung, die Sprühwirbelschichttrocknung, die Walzentrocknung und die Bandtrocknung, weniger bevorzugte aber auch anwendbare Verfahren sind die Gefriertrocknung und die Gefrierkonzentrierung.

Die durch Trocknung aus wäßriger Lösung erfindungsgemäß erhältlichen Polymerisatpulver sind in Alkoholen, Wasser und deren Mischungen gut löslich.

Ein besonderer Vorteil der erfindungsgemäßen Polymere besteht darin, daß sie bei pH-Werten im Bereich von 5,5 bis 7 bereits voll wasserlöslich sind, d.h. Haar und Kopfhaut werden nicht durch Alkalinität geschädigt. Durch die gute Wasserlöslichkeit können auch rein wasserbasierende Formulierungen hergestellt werden, was hinsichtlich der Umweltverträglichkeit besonders vorteilhaft ist. Die erfindungsgemäßen Polymere weisen gute festigende Eigenschaften bei gleichzeitig gutem Trocknungsverhalten und geringer Klebrigkeit auf und eignen sich daher besonders zum Einsatz als Filmbildner in Haarfestigungsmitteln wie Haarsprays, Haarlacken, Festigerlotionen oder Haarschäume oder Haargele.

### Beispiele

Die Trübung der wäßrigen Copolymerisat-Lösungen wurde durch nephelometrische Trübungsmessung bestimmt (modifizierte Methode nach DIN 38 404). Bei dieser Meßmethode wird photometrisch die Streuung von Licht nach Durchstrahlen der Meßlösung ermittelt, wobei diese Streuung durch die Wechselwirkung zwischen den Lichtstrahlen und den Partikeln oder Tröpfchen in der Lösung, deren Anzahl und Größe den Grad der Trübung ausmachen, bestimmt wird. Als Meßgröße dient die nephelometrische Trübungseinheit (NTU), welche bei 25°C und in 10-gew.-%iger wäßriger Lösung gemessen und durch Eichung auf der Basis von Formazin als künstlichem Trübungsmittel festgelegt wird. Je höher der NTU-Wert, desto trüber ist die Lösung.

### Herstellung der Polymere

### Beispiel 1

Es wurden zunächst die folgenden Lösungen vorbereitet:
1. Lösung 1, bestehend aus 150 g Ethylacrylat, 250 g iso-Butylmethacrylat und g Isopropanol.
2. Lösung 2, bestehend aus 100 g 2-Acrylamido-2-methylpropansulfonsäure, 17 g Natriumhydroxyd und 100 g Wasser.
3. Monomerzulauf, bestehend aus Lösung 1 und Lösung 2.
4. Starterzulauf, bestehend aus 8 g t-Butylperoxypivalat, gelöst in 150 g Isopropanol.
5. Vorlage, bestehend aus 90 g Monomerzulauf, 200 g Isopropanol und 0,5 g t-Butylperoxypivalat.

Die Vorlage wurde in einer Stickstoffatmosphäre bei einem Druck von 1,5 bar auf 78°C erwärmt. Nach dem Anspringen der Polymerisation, erkennbar an der exothermen Reaktion, wurde gleichzeitig mit der Zugabe des Starterzulaufs und des restlichen Monomerzulaufs begonnen, wobei der Monomerzulauf während einer Zeitspanne von 8 Stunden zugegeben wurde, der Starterzulauf während einer Zeitspanne von 16 Stunden. Anschließend wurde die Polymerisationsmischung noch 3 Stunden bei einer Temperatur von 78°C gehalten.

Danach wurde der Lösungsmittelaustausch durch Zugabe von Wasser und Entfernen des Isopropanols durch Wasserdampfdestillation vorgenommen.

Die erhaltene wäßrige Polymerlösung wies einen Feststoffgehalt von 30 Gew.-% auf. Der K-Wert (gemessen 1 % in N-Methylpyrrolidon) betrug 33,7. Beim Verdünnen der Lösung mit Wasser auf 10 % Feststoffgehalt wurde eine Lösung mit NTU-Werten von 30 erhalten, beim entsprechenden Verdünnen mit Ethanol auf 10 % Feststoffgehalt betrug der NTU-Wert der Lösung 15.

### Beispiel 2

Die Herstellung erfolgte analog Beispiel 1, jedoch wurde eine Lösung 1 verwendet, die als Monomere 150 g Ethylacrylat und 250 g tert.-Butylacrylat enthielt. Der K-Wert betrug 26,5 (gemessen 1 %ig in N-Methylpyrrolidon). Der NTU-Wert einer 5 gew.-%igen wäßrigen Lösung betrug 12,8.

### Beispiel 3

Die Herstellung erfolgte analog Beispiel 1. Lösung 1 enthielt 350 g tert.-Butylacrylat und 100 g N-Vinylpyrrolidon als Monomere. Lösung 2 enthielt 50 g 2-Acrylamido-2-methyl-propansulfonsäure und 8,5 g NaOH. Der K-Wert betrug 24,5, der NTU-Wert einer 5 gew.-%igen wäßrigen Lösung 16.
- Formulierungsbeispiel 1:: Pump-Spray, normale Festigung
5 Gew.-% Polymer von Beispiel 2
95 Gew.-% Wasser
q.s. Konservierungsmittel
q.s. Parfüm und Lösungsvermittler
(qs.: nach Bedarf)
- Formulierungsbeispiel 2:: Pump-Spray, starke Festigung
10 Gew.-% Polymer von Beispiel 2
90 Gew.-% Wasser
q.s. Konservierungsmittel
q.s. Parfüm und Lösungsvermittler
- Formulierungsbeispiel 3:: Haarspray ohne Ethanol, normale Festigung
5 Gew.-% Polymer von Beispiel 2
65 Gew.-% Wasser
30 Gew.-% Dimethylether (DME)
q.s. Parfüm und Lösungsvermittler
- Formulierungsbeispiel 4:: Haarlotion, normale Festigung
4 Gew.-% Polymer von Beispiel 2
86 Gew.-% Wasser
q.s. Konservierungsmittel
q.s. Parfüm und Lösungsvermittler

Die Polymere gemäß Beispiel 2 lassen sich sowohl als rein wäßrige Pumpsprays, als auch als wäßrige Aerosolsprays mit Dimethylether als Treibgas formulieren. Die Formulierung einer wäßrigen Haarlotion ist in Beispiel 4 beschrieben. Auf dem Haar wirken die erfindungsgemäßen Polymere normal bis stark festigend. Die Wasseraufnahmebereitschaft der behandelten Haare ist gering, wodurch die Frisur einen längeren Halt bekommt und weniger klebt.

Weiterhin zeichnen sich die erfindungsgemäßen Haarfestigungs- und Haarpflegemittel dadurch aus, daß sie das Haar praktisch nicht verkleben und es gut auskämmbar bleibt. Die behandelten Haare zeigen ein natürliches Aussehen. Der mit erfindungsgemäßen Mitteln erzielte Steifeffekt der Haare ist durchweg gut.

## Patentansprüche

1. Für den Einsatz in wäßrigen Haarfestigungsmitteln geeignete Polymere, erhältlich durch radikalisch initiierte Lösungspolymerisation eines Monomerengemischs aus
A) 50 - 90 Gew.-% eines oder mehrerer Monomeren aus der Gruppe bestehend aus C₁-C₁₈-Alkylestern der Acrylsäure oder der Methacrylsäure und Vinylestern von gesättigten C₂-C₁₀-Monocarbonsäuren,
B) 10 - 25 Gew.-% eines sulfonsäuregruppenhaltigen Vinylmonomeren, und
C) 0 - 40 Gew.-% eines weiteren Monomeren aus der Gruppe bestehend aus Acrylsäure, Methacrylsäure, Acrylamid, Methacrylamid, C₃-C₈-N-Alkylacrylamid, N,N-Dimethylacrylamid, N-Vinylpyrrolidon und N-Vinylcaprolactam.

2. Polymere nach Anspruch 1, wobei die Sulfonsäuregruppen der Monomere B) teilweise oder ganz als Salze anorganischer Basen oder organischer C₁-C₈-Stickstoffbasen vorliegen.

3. Polymere nach Anspruch 1 oder 2, erhältlich aus einer Monomerenmischung, bestehend aus
A) 70 bis 85 Gew.-% eines oder mehrerer C₁-C₈-Alkylester der Acrylsäure oder der Methacrylsäure,
B) 10 bis 20 Gew.-% 2-Acrylamido-2-methyl-propansulfonsäure, und
C) 0 bis 25 Gew.-% N-Vinylpyrrolidon.

4. Haarfestigungsmittel, enthaltend als Filmbildner Polymere gemäß einem der Ansprüche 1 bis 3.

5. Haarfestigungsmittel nach Anspruch 4, enthaltend als Lösungsmittel Wasser.
